Europäisches Patentamt

European Patent Office

Office européen des brevets

(11) Publication number: **0 285 159**
**A1**

## (12) EUROPEAN PATENT APPLICATION

(21) Application number: 88105269.0

(22) Date of filing: 31.03.88

(51) Int. Cl.⁴: **C12P 21/00** , C07K 15/00 ,
C12N 15/00 , G01N 33/68 ,
G01N 33/577 , //C12N5/00,
(C12P21/00,C12R1:91)

(30) Priority: 31.03.87 JP 78356/87

(43) Date of publication of application:
05.10.88 Bulletin 88/40

(84) Designated Contracting States:
AT BE CH DE ES FR GB GR IT LI LU NL SE

(71) Applicant: **Suntory Limited**
**1-40 Dojimahama 2-chome Kita-ku**
**Osaka-shi Osaka-fu 530(JP)**

(72) Inventor: **Yachi, Akira**
**2-31, Minami 14 Jo Nishi 14-chome**
**Chuo-ku Sapporo-shi Hokkaido(JP)**
Inventor: **Imai, Kohzoh**
**Kita 16 Jo Higashi 2-chome**
**Higashi-ku Sapporo-shi Hokkaido(JP)**
Inventor: **Yamashita, Takafumi**
**6-1, Tonden 5 Jo 3-chome**
**Kita-ku Sapporo-shi Hokkaido(JP)**

(74) Representative: **Hansen, Bernd, Dr.rer.nat. et**
**al**
**Hoffmann, Eitle & Partner Patentanwälte**
**Arabellastrasse 4 Postfach 81 04 20**
**D-8000 München 81(DE)**

(54) Human amyloid related protein monoclonal antibody.

(57) A monoclonal antibody which specifically recognizes a human amloid related protein. Especially, the antibody recognizes an area of amyloid deposition in secondary amyloidosis or an area of amyloid deposition in senile maculae in the brain of a patient with Alzheimer's dementia.

EP 0 285 159 A1

# HUMAN AMYLOID RELATED PROTEIN MONOCLONAL ANTIBODY

The present invention relates to a human amyloid related protein monoclonal antibody and uses thereof. More particularly, the present invention relates to a monoclonal antibody produced from the hybridoma obtained by fusion between myeloma cells and the spleen cells of a mammalian animal immunized with amyloid protein extracted from the tissues of organs of a patient with amyloidosis. The present invention also relates to a method of diagnosing secondary amyloidosis or Alzheimer's dementia.

Amyloidosis is a metabolic disease characterized by the deposition of amyloid protein in various organs of the body. Histologically, amyloid protein has a glass-like structure that is homogeneously eosinophilic in hematoxylin-eosin staining, and also has an ability to effect polarization of green light when stained with Congo red (Glenner, G. G. et al, J. Histochem. Cytochem., 22, 1141 - 1158, 1974). It has also been shown that amyloid protein microscopically has a fibrous structure with fiber width of 50 - 70 Å , and has a $\beta$-pleated sheet structure in X-ray diffraction analysis (Eomes, E. D. and Glenner, G. G., J. Histochem. Cytochem., 16, 673 - 677, 1968 and Cooper, J. H., Lab. Invest., 31, 232 - 238, 1974). Amyloid protein having these characteristics is not chemically a single substance and has been classified as being of various types. Although the accumulation of knowledge about the properties of amyloid protein is increasing gradually, amyloid A (AA) protein is the only type of amyloid protein that has been satisfactorily characterized (Benditt, E. P. et al., FEBS Lett., 19, 169 - 173, 1971 and Glenner, G. G., N. Engl. J. Med., 302, 1283 - 1292, 1980) and the chemistry of other types is scarcely known. Furthermore, a recently reported case of amyloid deposition in senile maculae in the brain of a patient suffering from Alzheimer's dementia has drawn researchers' attention in regard to the cause of this phenomenon and its pathology. However, systematic investigation of the similarities or dissimilarities of various types of amyloid protein has just begun, as has the study of amyloid related proteins occurring in tissues or blood at their molecular level. Monoclonal antibodies to naturally occurring amyloid proteins have been unknown too.

An object, therefore, of the present invention is to obtain a monoclonal antibody that binds specifically to a human amyloid related protein.

Another object of the present invention is to use this monoclonal antibody in diagnosis of amyloidosis, in particular, secondary amyloidosis, and also in diagnosis of Alzheimer's dementia.

The monoclonal antibody of the present invention is produced by the following basic steps: immunizing a mammal (preferably a mouse) with human amyloid protein that has been extracted from an autoptic material of a patient with amyloidosis; isolating antibody-producing cells from the animal; fusing these cells with tumor cells, such as myeloma cells, to obtain hybridomas that produce the desired antibody; and cloning and cultivating the hybridomas.

The process for preparing the monoclonal antibody of the present invention is hereunder described more specifically.

a) Preparation of antibody-producing cells

The production of desired hybridomas starts with immunizing or sensitizing a mammal with the human amyloid protein extracted in the manner already described. Any common mammals may be used such as mouse, rat, rabbit, guinea pig, etc. For instance, a mouse may be immunized by intraperitoneal or subcutaneous inoculation of the extracted human amyloid protein as an antigen. The inoculation consists of several applications, at 1 - 2 weeks intervals, of a suspension of a mixture of 10 - 30 μg of the antigen per mouse with an equal amount of an adjuvant. The final immunization is effected by intravenous or intraperitoneal injection of the adjuvant-free antigen in an amount of 50 - 150 μg/mouse. After 3 - 4 days, the spleen is extracted from the animal and subsequently processed to make antibody-producing cells. Human amyloid protein to be used is extracted from the tissue of an organ, preferably the kidney, of a patient suffering from amyloidosis.

b) Preparation of myeloma cells

There is no particular limitation in respect of the myeloma cells to be employed and cell lines from animals such as mouse, rat, rabbit and human are usable. Mouse myeloma cells are usually employed. Generally, cell lines of tumor such as myeloma having an appropriate selection marker such as hypoxanthine-guaninephosphoribosyltransferase deficiency (HGPRT⁻) or thymidine kinase deficiency (TK⁻) are provided, as exemplified by P-3-X63-Ag8, P3-X63-Ag8-U1, SP20-Ag14 and X63-Ag8-6.5.3. Such tumor cells are 8-azaguanine resistant cell lines and are unable to grow in a HAT medium.

## c) Cell fusion

Common media may be used, such as Eagles minimum essential medium (MEM), Dulbecco's modified MEM and Roswell Park Memorial Institute (RPMI) 1640 medium. To the medium, 10% CS (calf serum), 5% FCS (fetal calf serum) + 5% CS, or 10% FCS is added.

For the ordinary maintenance of parent cells, any one of the media listed above may be used but for the preparation of hybridomas, 10% FCS is desirably added. Cell fusion is performed on a 1:5 - 1:10 mixture of parent cells (tumor cells such as myeloma cells) and immunized or sensitized spleen cells (antibody-producing cells) in the presence of a fusion promoter. Illustrative fusion promoters are HVJ (Hemagglutinating Virus of Japan) and polyethylene glycol (PEG). The use of about 30 - 50% PEG 1500 is particularly preferred.

## d) HAT selection of hybridomas

The hybridomas obtained by cell fusion are appropriately diluted with a medium such as 20% FCS containing RPMI 1640 and the dilution is transferred to a microculture plate (usually having 96 wells) at a concentration of $10^5$ -$10^6$ cells per 100 $\mu$l/well. A HAT selection medium is added to each well and cultivation is performed with the medium replaced by a fresh one at given intervals, usually 1 - 2 day intervals. If an 8-azaguanine resistant strain is used as myeloma cells, unfused myeloma cells and myeloma-myeloma hybrid cells will die in about 10 days in a HAT medium. Spleen cells (splenocytes) are normal cells with a limited life span and are unable to live for more than 2 weeks in vitro. Therefore, it may safely be concluded that all of the cells that grow after about 10 - 14 days of cultivation are hybridomas between spleen cells and myeloma cells.

## e) Screening of hybridomas

Screening is preferably done by the ELISA (enzyme-linked immunosorbent assay) method or indirect immunoperoxidase method using the supernatant of culture in the wells where the growth of hybridomas occurred. Hybridoma clones that secrete the desired antibody are selected.

## f) Cloning

Each well might contain more than one kind of hybridomas that produce different antibodies but a homogeneous hybridoma which produces the desired monoclonal antibody can be finally obtained by performing cloning by a suitable method such as limiting dilution.

## g) Obtaining monoclonal antibody

The hybridomas obtained in step f) are cultivated either in a culture container (in vitro) or in the body of an animal (in vivo). In in vitro cultivation, one of the common media listed in c) may be used after supplementation with CS or FCS. After cultivation in the medium for 3 - 5 days, the desired antibody can be recovered from the culture supernatant. In in vivo cultivation, a mineral oil such as pristane (2,6,10,14-tetramethylpentadecane) is administered intraperitoneally to an animal which is syngeneic to the myeloma cells. After at least 1 week from the pristane treatment, the hybridomas are inoculated intraperitoneally and ascitic fluid accumulated in 7 - 14 days is collected. The desired antibody can be recovered from this fluid.

The so obtained monoclonal antibody can be utilized in assay or detection of human amyloid related proteins, as well as in diagnosis of diseases such as amyloidosis (especially secondary amyloidosis) and Alzheimer's dementia that are characterized by the deposition of amyloid protein in tissues.

The following examples are provided for the purpose of further illustrating the present invention but are in no way to be taken as limiting.

## Example 1 Preparation of Monoclonal Antibody

### a) Preparation of antigen

An autoptic kidney from a patient suffering from chronic arthritis, who was clinically diagnosed to have renal insufficiency with secondary systemic amyloidosis complications, was used as a material from which amyloid protein was to be extracted. Amyloid protein was extracted by the distilled water extraction method of Pras, et al. (J. Clin. Invet., 47, 924 - 933, 1968) as follows. The tissue of the kidney (wet wt. 20 g) was homogenized with 400 ml of a cold physiological saline solution and centrifuged. The pellet was washed with an equal volume of a cold physiological saline solution and subjected to repeated centrifugation until the protein concentration (OD 280 nm) of the supernatant decreased to 0.075 or below. The resulting pellet was homogenized in 200 ml of distilled water and centrifuged 4 times. The supernatants from the respective cycles were pooled and freeze-dried to obtain 200 mg of crude amyloid protein.

b) Immunization of animal

A portion (1 mg) of the crude amyloid protein was suspended in Freund's complete adjuvant (FCA) and administered subcutaneously to 7-week old BALB/c mice (Klea Japan, Inc.). After 2 weeks, the same volume of crude amyloid protein as that used in the initial immunization was suspended in FCA and administered subcutaneously to the mice as a booster. Two weeks after the second injection, 2 mg of crude amyloid protein was dissolved in distilled water and administered intraperitoneally to the animals to effect final immunization.

c) Cell fusion

Three days after the final immunization, spleen was extracted from the mice, minced and filtered through a stainless steel mesh to obtain spleen cells. Cell fusion was effected by mixing the spleen cells with mouse myeloma cells (X63-Ag8-6.5.3) in the presence of 50% polyethylene glycol 1540 (product of BDH). The fused cells were suspended in 10% FCS-RPMI at a concentration of about $1 \times 10^6$ cells/ml (calculated on the basis of the starting myeloma cells) and distributed on a 96-well microculture plate in portions of 100 $\mu$l per well.

After one day, 100 $\mu$l of a HAT medium was placed into each well and thereafter half its volume was replaced by a fresh HAT medium at 3-day intervals. In about 5 days, a growth of hybridomas was visible in some wells and, in 2 weeks, hybridomas were found to be growing in almost all wells.

d) Selection and cloning of hybridomas

The autoptic kidney from which crude amyloid protein was extracted in a) was fixed with 10% formalin, embedded in paraffin, sliced to 4 - 5 $\mu$m thick sections, and stained with hematoxylin-eosin or with Congo red to determine the locations at which amyloid protein was deposited. Thereafter, selection of active hybridomas was performed on the slices using the culture supernatant by the indirect immunoperoxidase method (Endo and Imai, Sapporo Igaku Zasshi, 54, 393 - 410, 1985) in accordance with the following procedure: the tissue sections were deparaffinized and the endogenous peroxidase activity was removed with 0.6% $H_2O_2$ in methanol; subsequently, in order to prevent non-specific adsorption of a second antibody (i.e. antibody to the monoclonal antibody) on the sections, they were reacted with normal rabbit serum, followed by reaction with the monoclonal antibody as a first antibody; reaction was then continued using peroxidase-labelled rabbit anti-mouse immunoglobulin (DAKO, Denmark) as the second antibody. Color reaction was effected with 3,3'-diaminobenzidine (Tokyo Kasei K.K.) and nuclear staining was conducted with 1% Methyl Green.

The supernatants of 10 hybridoma cultures showed highly specific reaction for the locations of amyloid deposition. Cloning was again performed by the limiting dilution method with respect to said cultures to obtain a single hybridoma clone.

e) Antibody recovery and identification of its subclass

The hybridoma cloned in d) was cultivated on a large scale in a cell culture flask (Corning 25110, USA) or propagated in the abdominal cavity of BALB/c mice (Klea Japan, Inc.). The culture supernatant or ascitic fluid was salted out twice with 50% ammonium sulfate and dialyzed against PBS for purification. Using rabbit anti-mouse $IgG_1$, $IgG_{2a}$, $IgG_{2b}$, $IgG_3$ and IgM (Miles, USA), the Ig subclass of the purified monoclonal antibody was determined by Ouchterlony method. It was found to be of subclass $IgG_1$ and designated as AM34. The AM34 producing hybridoma was named HY-AM34.

Example 2 Antibody Specificity

a) Absorption of AM34 by crude amyloid protein

The specificity of monoclonal antibody AM34 was checked on tissue sections. Before reaction on tissue sections, the monoclonal antibody was mixed with crude amyloid protein, and subjected to reaction at 37°C for 30 minutes. The mixture was then centrifuged. Using the supernatant as a first antibody, absorption of the monoclonal antibody by crude amyloid protein was examined by the indirect immunoperoxidase staining. Autoptic kidney sections prepared as described in Example 1, d) were used as the tissue sections. In this absorption test, reaction of the monoclonal antibody AM34 with the area of amyloid deposition completely disappeared.

b) Inhibition by rabbit anti-human amyloid A polyclonal antibody

Inhibition towards the reactivity of AM34 by rabbit anti-human amyloid A (AA) polyclonal antibody was checked on the autoptic sections described above by the indirect immunoperoxidase method. Before performing the indirect immunperoxidase method, the autoptic sections were pretreated at 37°C for 30 minutes with rabbit anti-human amyloid A (AA) serum (Kyowa Medex) which had been diluted to various concentrations. The reactivity of monoclonal antibody AM34 had a tendency to decrease slightly as the concentration of the polyclonal antibody used in the pre-treatment of the sections increased, indicating its ability to inhibit the reaction of AM34 to some extent. However, the increase in the concentration of the polyclonal antibody did not lead to complete loss of the reactivity of monoclonal antibody AM34.

c) Immunohistological reactivity of monoclonal antibody AM34 for various cases of amyloidosis

The reactivity of monoclonal antibody AM34 was examined by the indirect immunoperoxidase method on a total of 25 cases (36 tissue sections) of autopsy, surgical operation and biopsy that were conducted during the period 1967 - 1986. The 25 cases were: 12 cases (21 tissue sections) of primary amyloidosis or amyloidosis from multiple myeloma; 6 cases (8 tissue sections) of secondary amyloidosis; 2 cases of amyloidosis from familial polyneuropathy; 2 cases of Alzheimer's dementia involving senile maculae; 1 case of focal amyloidosis in spleen; 1 case of amyloidosis as a complication of endocrinoma; and 1 case of amyloidosis cutis. Each of the tissues under test was fixed in 10% formalin, embedded in paraffin, sliced into sections and stained with hematoxylin-eosin or with Congo red. With the samples stained with Congo red, polarization of green light was observed in all areas of amyloid protein deposition under a polarizing microscope.

Monoclonal antibody AM34 showed positive reactivity with the areas of amyloid deposition, especially in the cases of secondary amyloidosis. The fact that a positive reaction occurred in the areas that were presumably senile maculae in the brain tissue (hypocampus) of the cases of Alzheimer's dementia was also quite unexpected, even though only two such cases were tested. In the cases other than those of secondary amyloidosis, monoclonal antibody AM34 did not show any reactivity for the deposition of amyloid, the deposition area being stained only faintly, as far as can be seen from this analysis.

The above results suggest that a large amount of antigen epitopes corresponding to monoclonal antibody AM34 exist in the areas of amyloid deposition in secondary amyloidosis. It was also established (data not shown) by an immuno-electron microscopic analysis of an immunogen (kidney tissues) that monoclonal antibody AM34 reacts uniformly with amyloid fibers.

These results also suggest the potential use of monoclonal antibody AM34 in diagnosis of Alzheimer's dementia.

Example 3 Immunochemical Characterization of Corresponding Antigens

In order to determine the molecular weights of corresponding antigens for monoclonal antibody AM34, SDS-PAGE (sodium dodecylsulfate polyacrylamide gel electrophoresis) and Western blotting were performed using 15% gel in accordance with the method of Imai et al. (Cancer Res., 41, 1028 - 1033, 1981). Crude amyloid protein was dissolved in distilled water at a concentration of 3 mg/ml. Urea-treated protein (crude amyloid protein as treated with 8 M urea solution containing 1% sodium dodecylsulfate, 1% 2-mercaptoethanol, 0.01 M $H_3PO_4$ and 1 M Tris-Hcl, pH 6.5) and alkali-treated protein (crude amyloid protein as treated with 0.1 N NaOH for 2 hours at room temperature, then neutralized with 0.1 N HCl) were also each adjusted to a concentration of 3 mg/ml. A portion (50 µl) of each sample was subjected to SDS-PAGE, and transferred from the gel to a nitrocellulose membrane (pore size, 0.45 µm; Schleicher and Schuell, Germany) by the method of Watanabe et al. [Meneki Jikken Sosaho (Procedures of Immunological Experiments), ed. by S. Migita, 11, 3485 - 3489, 1982]. Antigen detection was then conducted by an enzyme-labelled antibody technique.

Monoclonal antibody AM34 showed specificity for the untreated crude amyloid protein and urea-treated amyloid protein at positions of 45 and 42 kilodaltons, respectively, with no visible difference occurring in molecular weight whether the condition was reducing or non-reducing. The monoclonal antibody did not show any reactivity for small molecule (8 and 9 kilodaltons) portions of AA protein treated either with urea or alkali. As a comparison, the same assay was conducted with monoclonal antibody KM268 that was prepared very recently as an antibody against synthetic AA peptide (gifted by courtesy of Prof. F. Uchino in Course of Pathology, Department of Medicine, Yamaguchi University). This antibody showed specificity for each of the antigen samples at a position corresponding to 9 kilodaltons but did not have any reactivity at positions of 45 and 42 kilodaltons where monoclonal antibody AM34 showed specificity. Reaction for these positions was not observed when polyclonal anti-AA protein antibody was used either.

In the analyses described above, the control antibodies used were monoclonal antibody H2H5 (IgG₁) prepared by the present inventors against human liver cancer cell line HuH7, polyclonal anti-AA protein antibody described in Example 2, b), and monoclonal antibody KM268 against synthetic AA protein.

Diagnosis of a specific disease type of amyloidosis has so far been conducted by immunohistological techniques using antisera against various types of amyloid protein. Being a homogeneous reagent, the monoclonal antibody of the present invention is potentially useful in identification of amyloid substances and in diagnosis of amyloidosis, notably secondary amyloidosis, as well as Alzheimer's dementia. Another advantage of this monoclonal antibody is that it permits the use of formalin-fixed and paraffin-embedded samples in immunohistological characterization.

The antigenic molecule that is recognized by the monoclonal antibody of the present invention is believed to be a new amyloid related protein that has been undetectable with the antibodies so far available. It is therefore anticipated that the monoclonal antibody of the present invention will be utilized in the future for diagnosis of new cases of previously unknown diseases.

## Claims

1. A monoclonal antibody which specifically recognizes a human amyloid related protein.

2. A monoclonal antibody according to Claim 1 wherein said protein has a molecular weight of approximately 42 or 45 kilodaltons as analyzed by SDS-PAGE or the Western blotting technique.

3. A monoclonal antibody according to Claim 1 or 2 which is of subclass IgG₁.

4. A monoclonal antibody according to any one of Claims 1 to 3 which recognizes an area of amyloid deposition in secondary amyloidosis or an area of amyloid deposition in senile maculae in the brain of a patient with Alzheimer's dementia.

5. A monoclonal antibody according to any one of Claims 1 to 4 which is obtained by cultivating hybridomas between myeloma cells and the spleen cells from a mammalian animal that has been sensitized with amyloid protein extracted from the tissue of an organ of a patient with amyloidosis.

6. A monoclonal antibody according to Claim 5 wherein the organ tissue is that of a kidney.

7. A monoclonal antibody according to Claim 5 wherein the mammalian animal is a mouse.

8. A monoclonal antibody according to any one of Claims 1 to 5 which is designated as AM34.

9. A method of diagnosing secondary amyloidosis or Alzheimer's dementia using a monoclonal antibody that specifically recognizes a human amyloid related protein.

10. A method according to Claim 9 wherein said monoclonal antibody is designated as AM34.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | US-A-4 666 829 (G.G. GLENNER et al.) <br> * Whole document * <br> --- | 1-10 | C 12 P 21/00 <br> C 07 K 15/00 <br> C 12 N 15/00 <br> G 01 N 33/68 <br> G 01 N 33/577// <br> C 12 N 5/00 <br> (C 12 P 21/00 <br> C 12 R 1:91 ) |
| X | CHEMICAL ABSTRACTS, vol. 107, no. 7, 17th August 1987, page 571, no. 57104u, Columbus, Ohio, US; T. YAMASHITA: "Immunological analysis of amyloid-related protein detected by a monoclonal antibody" & SAPPORO IGAKU ZASSHI 1987, 56(2), 219-31 <br> * Abstract * <br> --- | 1-10 | |
| X | BIOLOGICAL ABSTRACTS, vol. 83, no. 5, 1987, page AB-979, no. 48599, Philadelphia, PA, US; T. ISHII et al.: "A monoclonal antibody to amyloid in the brain of patients with Alzheimer's disease" & NEUROPATHOL APPL NEUROBIOL 12(5): 441-446. 1986 <br> * Abstract * <br> --- | 1-10 | |
| X | CHEMICAL ABSTRACTS, vol. 107, no. 7, 17th August 1987, page 555, no. 56927c, Columbus, Ohio, US; M.E. MARTIN et al.: "The physicochemical, antigenic, and functional heterogeneity of human serum amyloid A" & AMYLOIDOSIS. [PROC. INT. SYMP. AMYLOIDOSIS: DIS. COMPLEX], 4TH 1984 (PUB. 1986) 27-38 <br> * Abstract * <br> ---        -/- | 1-10 | **TECHNICAL FIELDS SEARCHED (Int. Cl.4)** <br><br> G 01 N <br> C 12 P |

The present search report has been drawn up for all claims

| Place of search | Date of completion of the search | Examiner |
|---|---|---|
| THE HAGUE | 07-07-1988 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | CHEMICAL ABSTRACTS, vol. 107, no. 3, 20th July 1987, page 309, no. 20061b, Columbus, Ohio, US; J. MARSHALL et al.: "In vivo radioimmunodetection of amyloid deposits in experimental amyloidosis" & AMYLOIDOSIS, [PROC. INT. SYMP. AMYLOIDOSIS: DIS. COMPLEX], 4TH 1984 (PUB. 1986), 163-74 * Abstract * | 1-10 | |
| X | CHEMICAL ABSTRACTS, vol. 106, no. 23, 8th June 1987, page 541, no. 194068d, Columbus, Ohio, US; R.P. LINKE et al.: "Ultrastructural identification of AA-type amyloid fibrils using polyclonal and monoclonal antibodies" & AMYLOIDOSIS. [PROC. INT. SYMP. AMYLOIDOSIS: DIS. COMPLEX], 4TH 1984 (PUB. 1986), 79-85 * Abstract * | 1-10 | |
| X | CHEMICAL ABSTRACTS, vol. 106, no. 3, 19th January 1987, page 432, no. 16581h, Columbus, Ohio, US; R.P. LINKE: "Two different sets of charge variants of amyloid fibril protein AA recognized by monoclonal antibodies" & PROTIDES BIOL. FLUIDS 1986, 34, 387-90 * Abstract * -/- | 1-10 | TECHNICAL FIELDS SEARCHED (Int. Cl.4) |

The present search report has been drawn up for all claims

| Place of search | Date of completion of the search | Examiner |
|---|---|---|
| THE HAGUE | 07-07-1988 | GRIFFITH G. |

EPO FORM 1503 03.82 (P0401)

European Patent
Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | BIOLOGICAL ABSTRACTS, vol. 82, no. 10, 1986, page AB-732, no. 94965, Philadelphia, PA, US; D. ALLSOP et al.: "Monoclonal antibodies raised against a subsequence of senile plaque core protein react with plaque cores, plaque periphery and cerebrovascular amyloid in Alzheimer's disease" & NEUROSCI. LETT., 68(2): 252-256, 1986 * Abstract * --- | 1-10 | |
| X | CHEMICAL ABSTRACTS, vol. 100, no. 15, 9th April 1984, page 274, no. 117361t, Columbus, Ohio, US; R.P. LINKE: "Monoclonal antibodies against amyloid fibril protein AA. Production, specificity, and use for immunohistochemical localization and classification of AA-type amyloidosis" & J. HISTOCHEM. CYTOCHEM. 1984, 32(3), 322-8 * Abstract * --- | 1-10 | |
| X | CHEMICAL ABSTRACTS, vol. 98, no. 3, 17th January 1983, page 387, no. 15183j, Columbus, Ohio, US; D.D. WOOD et al.: "An ELISA assay for murine amyloid A and serum amyloid A utilizing monoclonal antibodies" & J. IMMUNOL. METHODS 1982, 55(1), 19-26 * Abstract * ------ | 1-10 | |

TECHNICAL FIELDS
SEARCHED (Int. Cl.4)

The present search report has been drawn up for all claims

| Place of search | Date of completion of the search | Examiner |
|---|---|---|
| THE HAGUE | 07-07-1988 | GRIFFITH G. |

EPO FORM 1503 03.82 (P0401)